# EUROPEAN PATENT SPECIFICATION

(11) **EP 0 862 603 B1**
(45) Date of publication and mention of the grant of the patent: **19.01.2000**
(21) Application number: 96939872.6
(22) Date of filing: 21.11.1996
(51) Int. Cl.: C09K 7/02, C07H 15/04

(54) **WELL FLUID**
BOHRLOCHBEHANDLUNGSFLÜSSIGKEIT
FLUIDE DE FORAGE

(30) Priority: 22.11.1995 IT MI952422
(43) Date of publication of application: 09.09.1998
(73) Proprietor: LAMBERTI S.p.A., 21041 Albizzate (Varese) (IT)
(72) Inventor: NICORA, Luigi, I-21100 Varese (IT); GRAZIOSI, Marco, I-21048 Solbiate Arno (IT); PIROVANO, Pierangelo, I-21025 COMERIO (IT)
(74) Representative: Gervasi, Gemma, Dr.
(86) International application number: EP9605128
(87) International publication number: WO9719145

(56) References cited:
- EP-A- 0 681 016
- EP-A- 0 702 073
- EP-A- 0 728 826
- WO-A-94/14919
- WO-A-95/04592

## Description

### FIELD OF THE INVENTION

The invention concerns fluids which can be used in the search for subterranean reservoirs of hydrocarbons and water, in geognostic drilling, in civil engineering operations etc., improved as regards specific properties and with a low environmental impact, containing environmentally compatible additives. In particular the improvement in specific properties concerns the fluid loss and the fluid resistance to thermal aging.

The field of invention comprises, besides drilling and completion fluids, also all the water based systems used in well operations such as work-over, milling stimulation, fracturing, spotting fluids, cementing, etc. In the text the term "well fluid" will be used as reference to all this range of applications.

### PRIOR ART

The well fluids have a natural trend to lose the water phase through permeable rock formations met during drilling, because of pressure, which is generally higher inside the well than the pore pressure in the rock formation. In the course of the years various additives have been studied which, both in static and dynamic conditions, enable the building of a filter cake adhering to the well walls, having a thickness low enough not to limit the fluid circulation in the well and a permeability limited enough to reduce the fluid loss due to filtration.

Among the most known additives used in drilling fluids and in order to obtain the desired functional characteristics, with effect on fluid loss control, the following can be cited:
- water swellable clays such as bentonite, which generally require the use of other filtrate reducing agents;
- lignite, eventually oxidised, sulphonated, sulphomethylenated, treated with sodium hydroxide or with polyvalent metal salts such as Fe, Cr, Ti;
- lignosulphonates of Ca, Cr, Fe, Ti;
- natural polymers such as starch and its derivatives (carboxymethylated or hydroxyethylated starch), carboxymethylcellulose at different degrees of substitution and of molecular weight, PACs (polyanionic celluloses), polygalactomannans (e.g. guar) and its derivatives (e.g. carboxymethylated and hydroxypropylated), biopolymers such as xanthan gum, scleroglucan, succinoglycan, etc.;
- synthetic polymers such as homo and copolymers of acrylic acid and acrylamide, 2-acrylamido-2-methyl propane sulphonic acid, etc. and their water soluble salts.

The well fluids containing the above mentioned additives meet specific technical requirements; however because of the high variability of the rock formations, temperature and pressure conditions ever increasing with depth, it is often necessary to vary considerably the additive dosage or use different additives to obtain the desired effect.

As the additive or additives used generally also influence other characteristics (viscosity, lubrication, clay swelling inhibition, etc.), also these vary and often in a undesirable way. The correct fluid formulations therefore are a delicate compromise among the various properties and the fluid loss, which implies the alternative or complementary use of various additives and the careful search for the best dosage.

In the case of high dosage necessary for particularly permeable rock formations, or with particularly high pressures and temperatures, other drawbacks consist, for instance, in the high formulation cost, in biodegradability problems and in the polluting potential of the additives used and in the necessity to operate in confined spaces (e.g. on offshore platforms) and therefore materials are stocked in minimal quantities.

More recently, combinations of water soluble polymers and other components have been studied in order to improve specific properties.

In the US patent 4.900.457 (Shell Oil Company) A. S. Clarke-Sturman et al. describe a polysaccharide solution composition and its use as drilling fluid, characterized by the presence of water soluble polymers and alkaline formates. The claimed improvement consists in the resistance of the rheological properties after aging of the composition at high temperature, obtained through the addition of a sufficient quantity of formates. No evidence is reported for an eventual decrease of the fluid loss.

In the EP patent 541 606 (Henkel) H. Mueller et al. describe the use of alkylpolyglycosides as emulsifying surfactants for well fluids, containing a continuous or dispersed oil phase together with a water phase. These additives enable water-in-oil (invert) or oil-in-water (direct) emulsions to be obtained with good stability.

In the WO 95/04592 (S.E.P.P.I.C.) Lecocu-Michel discloses concentrated aqueous compositions comprising (i) more than 12% by weight of one or more alkylpolyglycosides, the fatty chain of each of said alkylpolyglycosides comprising at least 10 carbon atoms, and (ii) at least one amphoteric surfactant. Said compositions may be used for preparing formulations suitable for cosmetics, pharmaceutics, detergents, hygiene products and drilling compositions. The use of alkyl(poly)glycosides is required when it is necessary to emulsify two incompatible phases; no effect of filtrate reduction is reported, even in the presence of natural or synthetic polymers.

In the US patent 5.403.820 (O'Brien, Goins, Simpson & Associates Inc.) and in SPE/IADC 29404 T.O. Walker et al. describe drilling fluids characterized by the addition of water soluble alkylglycosides with alkyl chains containing between 1 to 4 carbon atoms and containing, among other things, additives for the fluid loss control.

The claimed improvement consists in the swelling reduction of the clays met by the fluid during drilling operations. This improvement is obtained provided that the alkyl radical of the alkylglycoside is able to maintain good water solubility and it therefore contains from 1 to 4 carbon atoms, preferably 1 carbon atom. In the above mentioned patent application no specific interaction between the alkylglycoside added and the other fluid components is reported. The concentration of alkylglycoside in the water phase must range from 35 to 65% in weight to be effective: in such ratios the component cannot be considered an additive any more but a base constituent of the fluid and therefore it directly determines economic aspects.

The above mentioned aspects stress the necessity of a research for more effective additives to be used in the fluid loss control and for the resistance of the mud to thermal aging.

### SUMMARY

The scope of this invention is to describe new well fluids with better resistance to fluid loss and temperature over a wide range of temperatures and pressures, obtainable more economically and with a lower environmental impact at the same performance.

Surprisingly we have found that water based well fluids containing a very low quantity of at least one alkyl(poly)glycoside (APG) with alkyl chain C₈ - C₂₆ and at least one water soluble polymer in the suitable ratios determine a better fluid loss control and a better resistance to thermal aging. The water soluble polymer can be of natural origin, or a modified natural polymer, or of synthetic origin; the alkyl(poly)glycoside consists of an aliphatic chain bound to a monosaccharide or to an oligosaccharide through an o-glycoside bond or of similar products as indicated by the formula (I). The APG quantity and its weight ratio to the water soluble polymer and the type of the water soluble polymer determine the characteristics of filtrate reduction and the resistance to thermal aging.

This invention therefore describes these fluids, the process for their preparation and their use as well fluids.

### BRIEF DESCRIPTION OF THE DRAWING

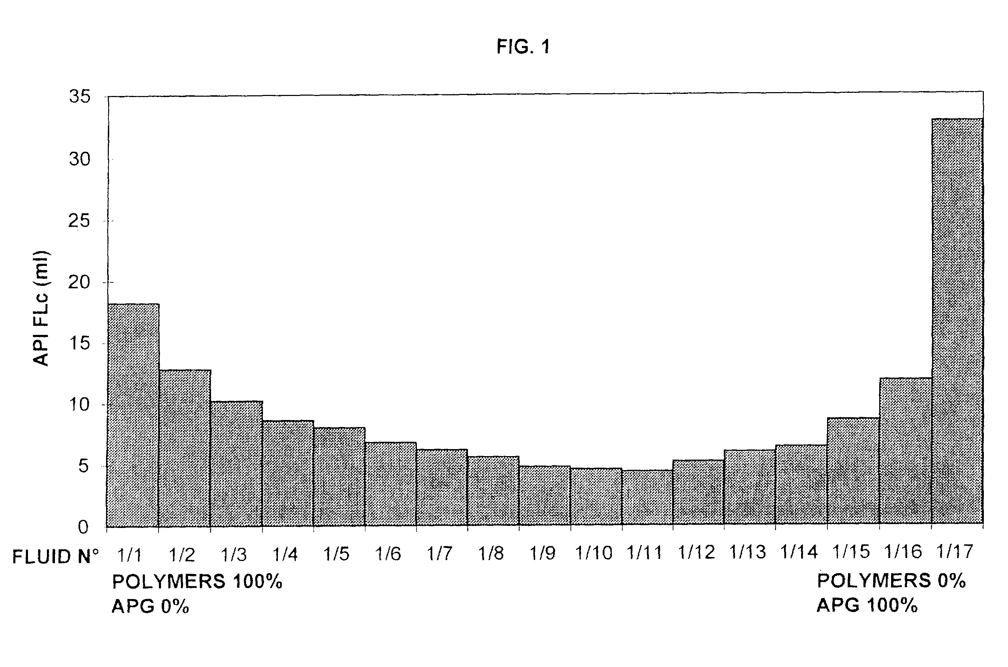

Figure 1 represents the loss of filtrate (API FLc) using combinations of water soluble polymers with APG in comparision with the use of only one of such substances.

### DETAILED DESCRIPTION OF THE INVENTION

The well fluids of this invention are based on a continuous water phase carrying components of varying nature with different functions, as known in the art (see e.g. G. R. Gray, H.C.H. Darley, W. F. Rogers, "Composition and Properties of Oil Well Drilling Fluids" 4th Edition, Gulf Publishing Co., Houston, Texas, U.S.A.,1980); these are generally weighting agents, viscosifiers, rheology modifiers, dispersants thinners, emulsifiers, lubricants, defoamers, biocides, pH modifiers, corrosion inhibitors, etc. Such fluids, in particular, also contain at least one filtrate reducer chosen among water soluble polymers of natural origin, eventually modified or of synthetic origin.

Examples of water soluble polymers suitable for the use according to this invention are the cellulose ethers, PACs, starch and its derivatives, polygalactomannans (e.g. guar) and derivatives, biopolymers of varying nature; homopolymers of acrylic acid, of acrylamide, of 2-acrylamido-2-methylpropanesulphonic acid and copolymers with the above monomers or of the above monomers with other acrylic or vinylic monomers and their water soluble salts. These polymers are characterized by such behaviour in solution as enables reduction of the fluid loss with respect to the base fluid without the water soluble polymer. Water soluble polymers particularly preferred are PACs and the carboxymethylcelluloses of various substitution degrees and molecular weight.

A detailed description of such water soluble polymers, which are commercially available from various sources, is reported on G. R. Gray, H.C.H. Darley, W. F. Rogers, "Composition and Properties of Oil Well Drilling Fluids" 4th Edition, Gulf Publishing Co., Houston, Texas, U.S.A., 1980. The concentrations of water soluble polymers normally used (expressed in weight/fluid weight) range from 0.02 to 5% and preferably from 0.05 to 3%.

Surprisingly it has now been found that the introduction in the well fluids of at least one alkyl(poly)glycoside with aliphatic chain containing from 8 to 26 C atoms, enables a better fluid loss control and a better resistance to thermal aging, in presence of water soluble polymers, even at very low dosages of this alkyl(poly)glycoside, and in particular from 0.001 to 3% in weight on the fluid weight, even in dynamic conditions and at high pressures and temperatures as required in many operations (i.e. up to 1000 atm and 150 °C).

This alkyl(poly)glycoside is chosen among the compounds represented by the following formula (I):

R-O-(A-O)_{X}-(G)_{Y}-(D)_{Z} (I)

where:
O represents an oxygen atom;
R represents an alkyl or alkenyl group having from 8 to 26 C atoms, linear or branched, unsubstituted or hydroxy substituted;
A represents an alkylene group having from 2 to 4 C atoms, linear or branched, or a bifunctional residue of a polyalcohol remaining after removing 2 hydroxyl groups on any 2 carbon atoms (e.g. a residue of sorbitol);
G represents a saccharide residue remaining after removing z hydrogen atoms from all the non glycosidic hydroxyl groups and removing the glycosidic hydroxyl group of a reducing sugar made of hexoses or/and pentoses, bound to A-O or to R-O (in the case x=0) through an o-glycosidic ether bond;
D represents an acyl residue of an organic acid, bound to an oxygen atom of the residue (G)_{Y} having formula 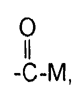 where M represents an alkyl or alkenyl chain having from 1 to 18 C atoms, linear or branched, unsubstituted or substituted by one or more groups such as OH, COOMt, SO₃Mt, -OPO₃Mt₂, NH₂, NR¹R², where R¹ and R² can be the same or different and represent alkyl chains having from 1 to 4 C atoms, linear or branched and Mt represents one hydrogen atom or a cation such as Li, Na, K, Cs, Ca, Mg, Fe, NH₄+;
x is a number from 0 to 10 which represents the average condensation degree of A, when A represents an alkylene group and is equal to 1 when A represents a bifunctional residue of a polyalcohol remaining after removing 2 hydroxyl groups;
y is a number from 1 to 10 which represents the average condensation degree of G;
z is a number from 0 to 10 which represents the average esterification degree of (G)_{Y}.

The compounds of formula (I) are known and widely described, whether concerning their synthesis and corresponding preparation methods, or for the physico- chemical and application properties, as e.g. reported on M. R. Porter, Handbook of Surfactants, Blackie Academic & Professional, London (2nd Ed. 1994) 202-210; F. A. Hughes and B. W. Lew, J. Am. Oil Chem. Soc. 1970 47, 162-167; F. Lomax, Specialty Chemicals, Jan-Feb. 1994, 21-24.

The compounds of formula (I) can be used in purified form or unpurified, as obtained from the synthesis. The introduction in the well fluid can be through direct addition of the compounds of formula (I), as they are, or in solution in suitable solvents (water, alcohols etc.) or as suspension in a suitable material. Alternatively it is possible to previously treat (i.e. mix or soak) other ingredients of the well fluid (e.g. water soluble polymers) with the compounds of formula (I).

The preferred compounds within this invention are those of formula (I) where:
R represents a linear alkyl group having from 10 to 26 C atoms, unsubstituted or hydroxy substituted;
x = 0, or = 1 when A is a bifunctional residue of sorbitol remaining after removing 2 hydroxyl groups;
G represents a glucose residue remaining after removing 1 hydrogen atom from the non glycosidic hydroxyl groups and removing the glycosidic hydroxyl group;
y is a number from 1 to 3;
z = 0, or = 1 when
   D = CO(CHOH)₂COOMt or
   D=COCH₂C(OH)(COOMt)CH₂COOMt or
   D =COCH₂CH(SO₃Mt)COOMt or
   D = COCH(SO₃Mt)CH₂COOMt, with Mt = H or Na.

Examples of the preferred compound of formula (I) are the alkyl(poly)glycosides not derivatized in which x = 0, G is a glucose residue, y = 1-2, z = 0 and R is a linear saturated not substituted alkyl group, having from 10 to 26 C atoms, as e.g. described in the patents US 3.219.656, US 3.547.828, US 3.839.318; other preferred compounds of formula (I) are the esters of the alkyl(poly)glycosides in which x = 0, G is a glucose residue, y = 1-2, z = 1 and D is a monoacyl residue of a bi- or tri-carboxylic acid, eventually sulphonated, in acid form or neutralized with Na and R is a linear saturated not substituted alkyl group having from 10 to 26 C atoms, as i.e. described in the patents EP 258814, EP 510564, EP 510565; other preferred compounds of formula (I) are glycosides in which R is a linear alkyl group having from 10 to 18 C atoms substituted with hydroxyl group in position 2, A is sorbitol group, x = 1, G is a glucose residue, y = 1-2, z = 0, as described in the patent EP 525494.

The preferred compounds of formula (I) are commercially available from various sources and all are essentially biodegradable.

For the preparation of the well fluids according to this invention, the combinations preferred are those formed by alkyl(poly)glycosides having alkyl chains from 10 to 26 C atoms and by a water soluble polymer chosen among carboxymethylcellulose, polyanionic cellulose (PAC), polygalactomannans (e.g. guar) and biopolymers (e.g. xanthan). The quantity of the compound of formula (I) to be introduced in the water well can vary, as specified above, from 0.001 to 3% by weight on the fluid.

In particular it is convenient to fix the water soluble polymer content in the drilling fluid and indicate the ratio in weight of the compound of formula (I) with respect to the water soluble polymer. In this way it results that, if the polymer concentration in the fluid is as previously indicated, this weight ratio can vary from 2 x 10⁻⁴ to 150 preferably, and conveniently from 10⁻³ to 10.

The fluids containing at least one water soluble polymer and at least one alkyl(poly)glycoside, in quantities and ratios as reported above, exhibit a significantly reduced fluid loss with respect to the same fluids not containing the component of formula (I) and have a better resistance to thermal aging, as can be seen in the examples to follow.

In particular it is possible to exploit the alkyl(poly)glycoside behaviour in combination with the water soluble polymer in order to effectively control the fluid loss at high pressures and temperatures (up to 1000 atm and 150 °C) where the use of only the water soluble polymer would require a much higher dosage to obtain the same performance.

Thanks to their characteristics, the well fluids according to this invention can be successfully employed in the search for hydrocarbons, water beds, in geognostic drilling and in civil engineering operations and in particular in drilling, completion, work-over, milling stimulation, fracturing and spotting fluids.

To better illustrate the invention, in the following examples is reported the effect of the addition of some types of formula (I) compounds using the same water soluble polymer, at various ratios in weight between formula (I) compounds and the water soluble polymer; the effect of the combination of the formula (I) compounds with different water soluble polymers is also reported and how such an effect is influenced by the nature of the fluid. For a better understanding of the experimental aspects and the importance of the fluid components, their preparation and evaluation methods of their properties will be first described.

The fluids used in the examples have been prepared according to the recipies reported for each example and the rheological and fluid loss characteristics have been measured using the apparatus and the methods described in "Standard Procedure for Field Testing Water-Based Drilling Fluids", API Recommended Practice 13B-1 (RP 13 B-1), First Edition, June 1, 1990, edited by American Petroleum Institute, 1220 L. Street, Northwest, Washington, DC 20005, U.S.A. In particular the rheological properties have been measured with a Direct Indicating Viscometer (Section 2) at a temperature of ca. 25°C.

Here the following abbreviations have been used: AV=apparent viscosity, PV= plastic viscosity, YP=yield point, BA=before aging, AA=after aging, n.d.= not determined. The fluid loss properties (FL) have been measured with the equipment and the procedures detailed in Section 3.

The meaning of "API FL" (FL) is the filtrate value equal to the volume taken in the first 30 minutes from the beginning of the test, obtained at room temperature (ca. 25°C) and at an applied pressure of 100 psi; the meaning of "API FL corrected" (FLc) is the filtrate value equal to the double of the volume taken between 7.5 minutes and 30 minutes from the beginning of the test, obtained under the same conditions.

The meaning of HPHT FL AA (high temperature/high pressure fluid loss test after aging) is the fluid loss volume obtained at a temperature of 120°C, with a difference in applied pressure of 500 psi, filtrate value equal to the volume taken in the first 30 minutes from the beginning of the test. The high temperature aging of the muds has been carried out in suitable heat aging cells in a roller-oven at 120°C for 16 hours, with the procedures generally known to the experts.

The synthetic sea water is prepared according to the specifications ASTM D 1141-75 (Standard Specifications for Substitute Ocean Water).

The commercial products used in the examples are the following:

| **PRODUCT** | **DESCRIPTION** | **SOURCE** |
|---|---|---|
| CEPAC REGULAR | Polyanionic cellulose | Lamberti S.p.A. - Italy |
| LAMPAC EXLO | Polyanionic cellulose | Lamberti S.p.A. - Italy |
| LAMPAC REGULAR | Polyanionic cellulose | Lamberti S.p.A. - Italy |
| CARBOCEL LV | Carboxymethylcellulose | Lamberti S.p.A. - Italy |
| CARBOCEL EHV | Carboxymethylcellulose | Lamberti S.p.A. - Italy |
| LAMGUM 200 | Guar flour | Lamberti S.p.A. - Italy |
| VISPLEX | Alluminium Magnesium Hydroxychloride (MMH-Mix Metal Hydroxide) | Schlumberger Dowell Drilling Fluids - France |
| FLOPLEX | Modified starch | Schlumberger Dowell Drilling Fluids - France |
| DEXTRID | Modified starch | Baroid Drilling Fluids Inc.-U.S.A. |
| POLY PLUS | Copolymer sodium acrylate-acrylamide in inverted emulsion (PHPA) | M -I Drilling Fluids-U.S.A. |
| Q BROXIN | Ferrochromolignosulphonate | Baroid Drilling Fluids Inc.-USA |
| IDVIS | Xanthan Gum | Schlumberger Dowell Drilling Fluids - France |

As alkyl(poly)glycosides (APG) have been used the following (described with reference to the formula (I)):

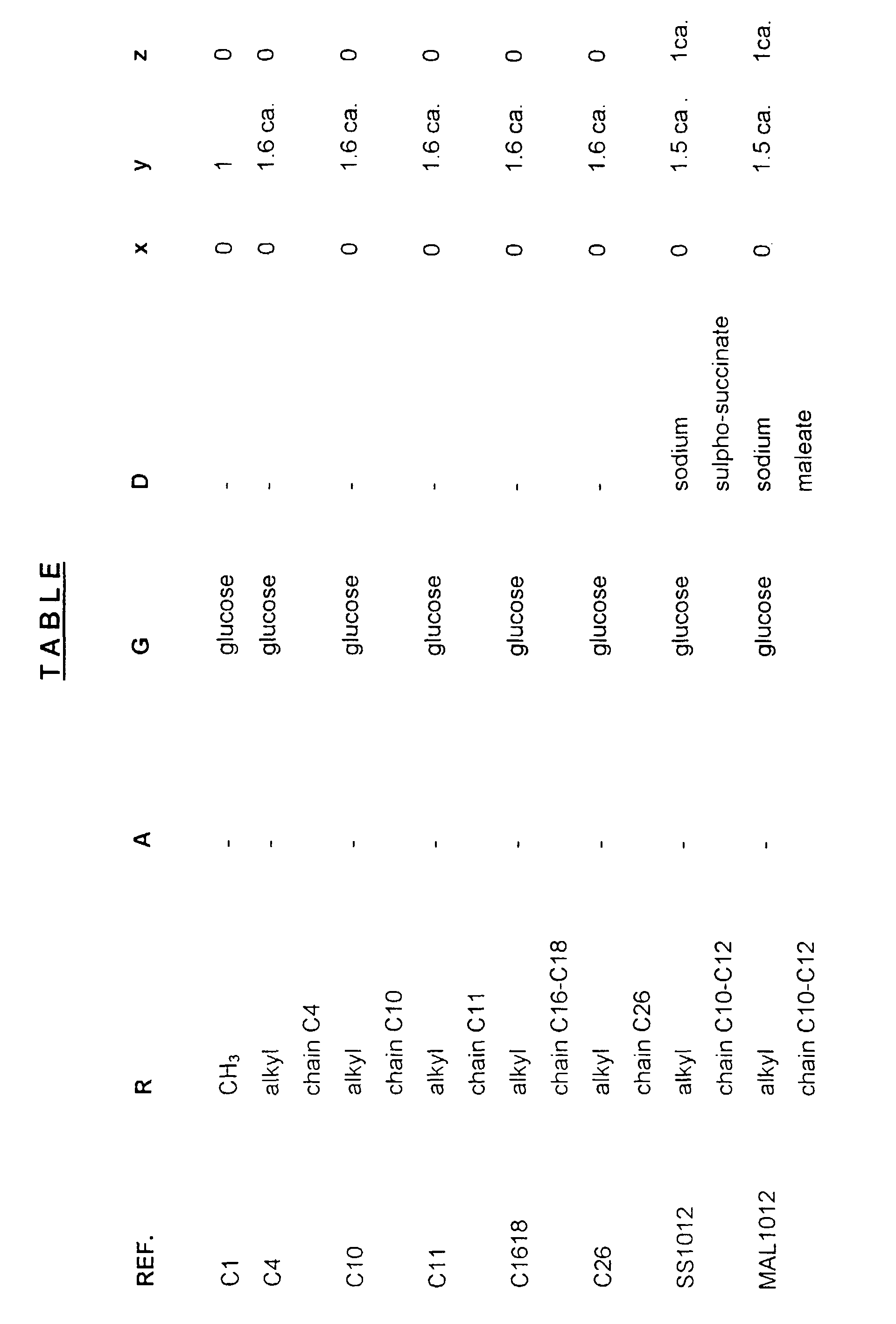

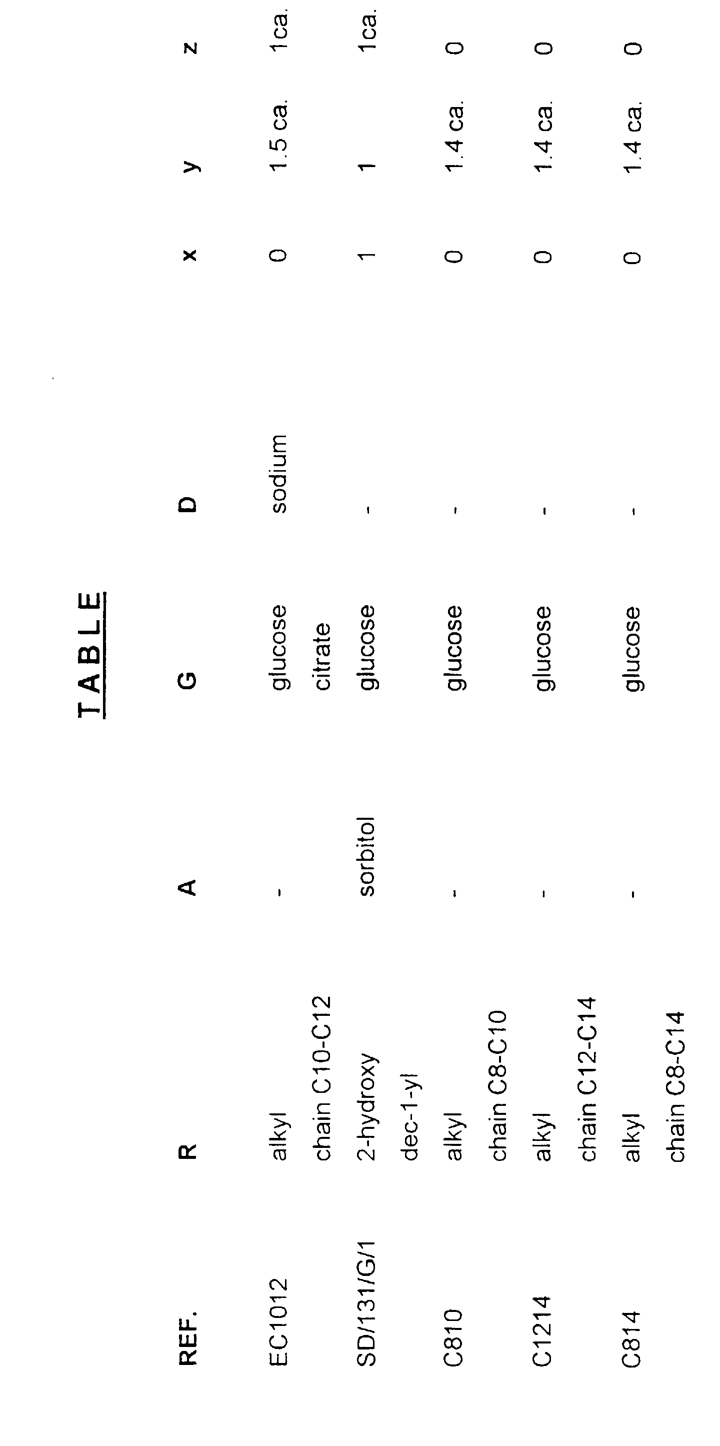

The compounds indicated with references C1 and C4 are not considered in this invention and are reported as comparison.

The water insoluble APG have been added to the fluid after dissolution in ethanol at a dry content of ca. 15% weight/alcohol weight. The quantities reported always refer to the effective content of APG.

Thanks to its characteristics the fluids according to this invention can be successfully used in drilling, completion, work-over, milling stimulation, fracturing and spotting fluids operations, concerning hydrocarbon or water beds research, in geognostic drilling and in civil engineering operations.

To illustrate but not to limit this invention the following examples are reported.

### EXAMPLE 1

A range of well fluids has been prepared having variable content of water soluble polymer and APG and comparing fluids without APG or water soluble polymer and the API filtrate loss corrected (FLc) has been determined as reported below.

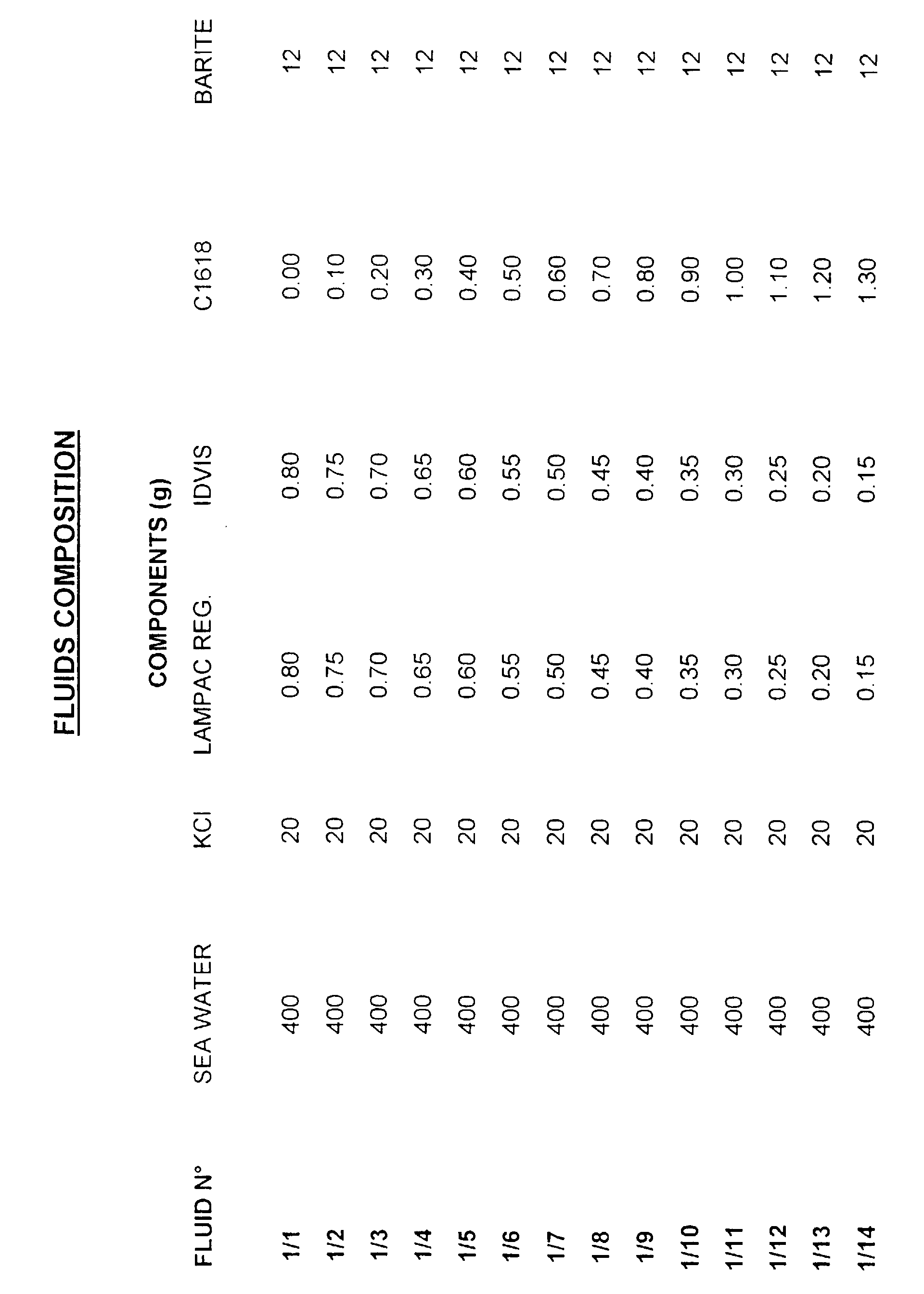

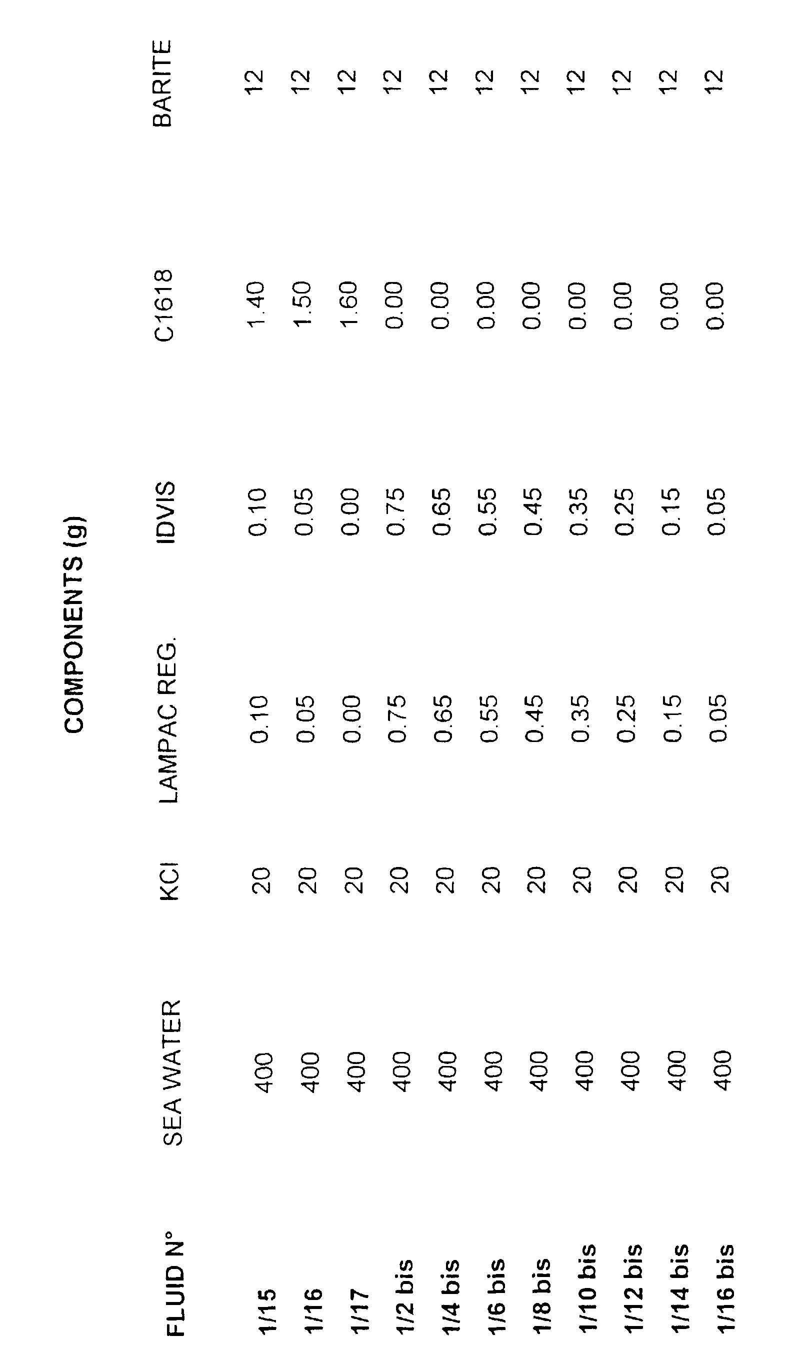

| **RESULTS OBTAINED** | | | |
|---|---|---|---|
| **FLUID N°** | API FLc (ml) | AV AA (cP) | %C1618 WEIGHT/WEIGHT |
| **1/1** | 18.2 | 18 | - |
| **1/2** | 12.8 | 18 | 0.023 |
| **1/3** | 10.2 | n.d. | 0.046 |
| **1/4** | 8.6 | 16 | 0.069 |
| **1/5** | 8.0 | n.d. | 0.092 |
| **1/6** | 6.8 | 14 | 0.115 |
| **1/7** | 6.2 | n.d. | 0.138 |
| **1/8** | 5.6 | 11 | 0.161 |
| **1/9** | 4.8 | n.d. | 0.184 |
| **1/10** | 4.6 | n.d. | 0.207 |
| **1/11** | 4.4 | n.d. | 0.231 |
| **1/12** | 5.2 | n.d. | 0.254 |
| **1/13** | 6.0 | n.d. | 0.277 |
| **1/14** | 6.4 | n.d. | 0.300 |
| **1/15** | 8.6 | n.d. | 0.323 |
| **1/16** | 11.8 | n.d. | 0.347 |
| **1/17** | 32.8 | n.d. | 0.370 |
| **1/2 bis** | 18.2 | n.d. | - |
| **1/4 bis** | 18.4 | n.d. | - |
| **1/6 bis** | 18.8 | n.d. | - |
| **1/8 bis** | 19.6 | n.d. | - |
| **1/10 bis** | 21.2 | n.d. | - |
| **1/12 bis** | 27.8 | n.d. | - |
| **1/14 bis** | 61.0 | n.d. | - |
| **1/16 bis** | 154.0 | n.d. | - |

From the above mentioned tests it follows that using combinations of water soluble polymers with APG in any ratio reduces the filtrate loss compared to the use of only one of said additives with the same total content, showing a synergic effect, as represented in figure 1.

Moreover, it is shown that a substitution of 15% of water soluble polymers with APG is able to reduce the fluid loss by approximately 50%.

### EXAMPLE 2

A range of well fluids has been prepared through addition of a constant minimum quantity of different types of alkyl(poly)glycosides to the soluble polymers present in the fluid (CEPAC REGULAR) and the reduction of the HPHT FL AA at 120°C has been determined.

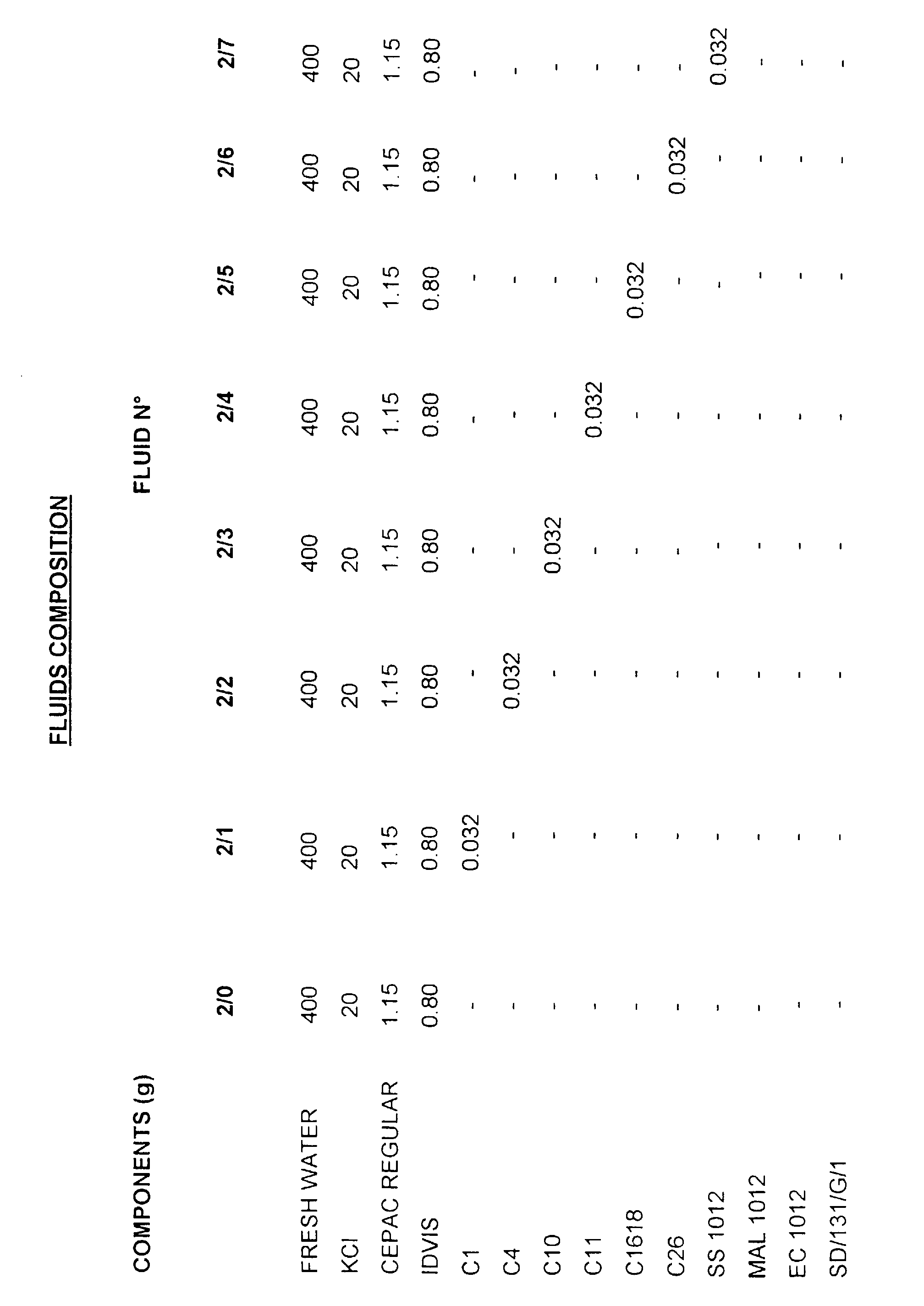

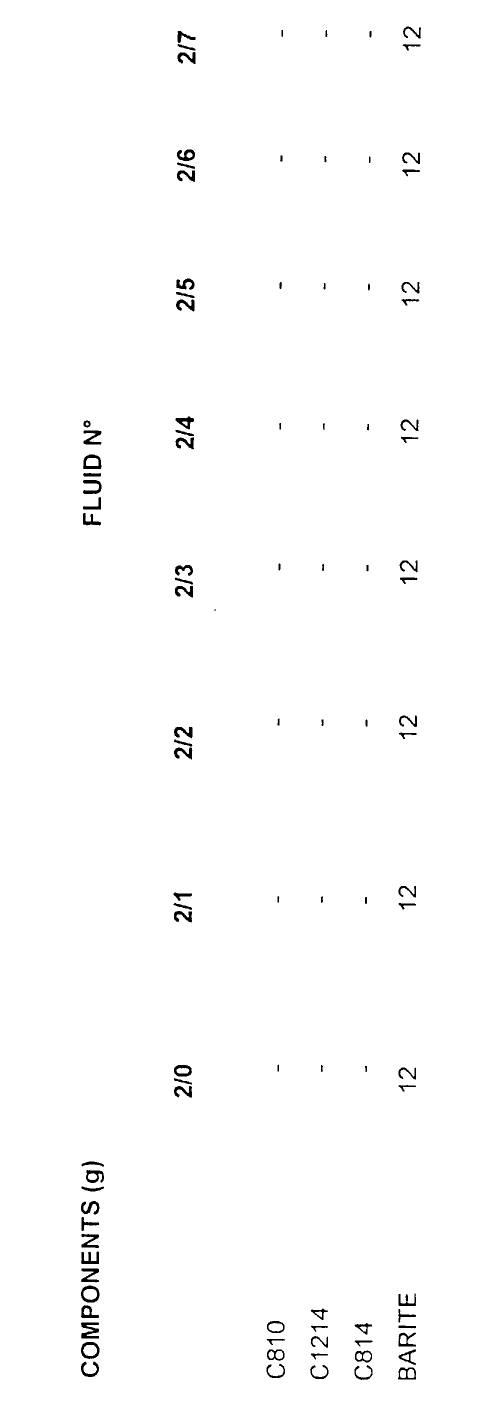

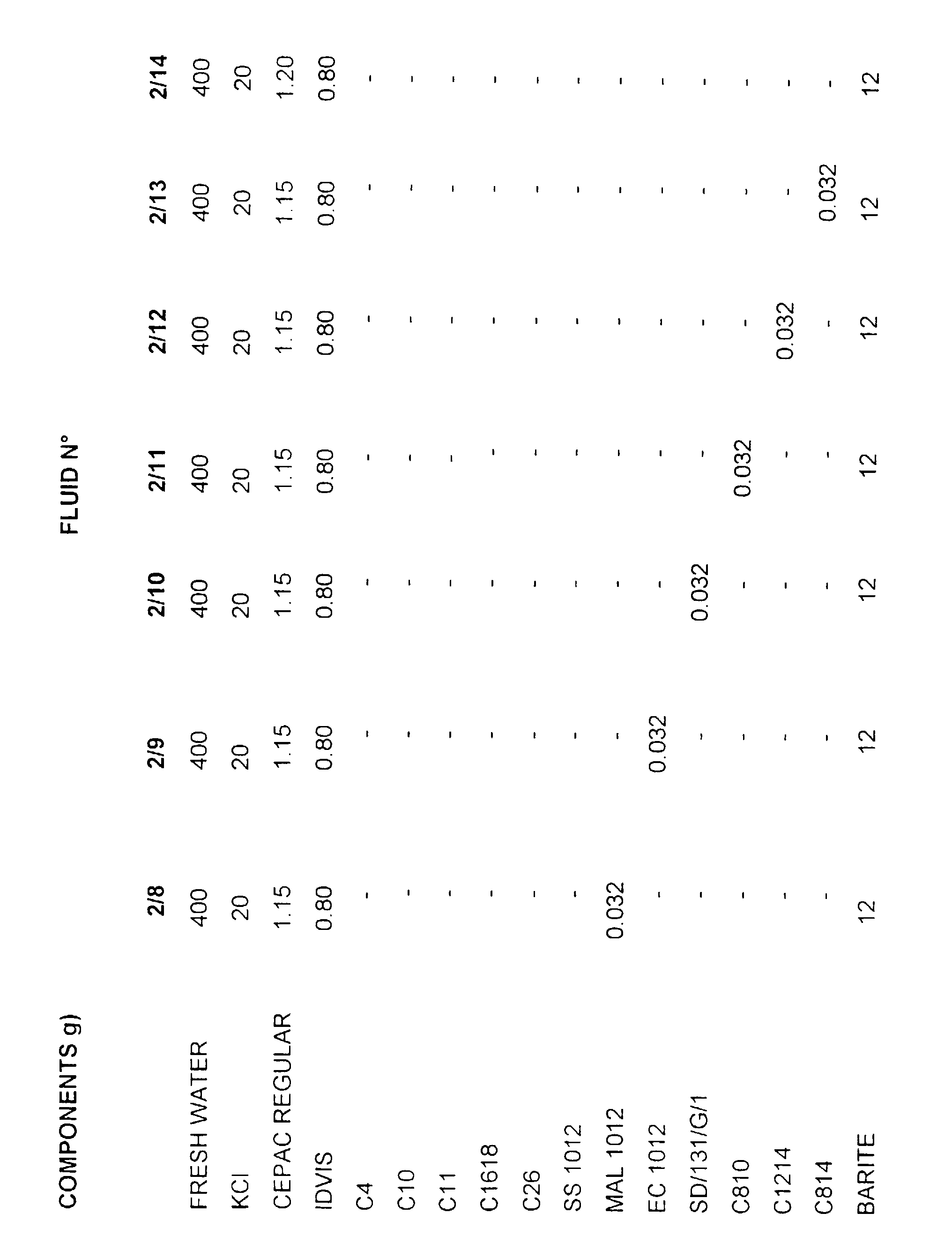

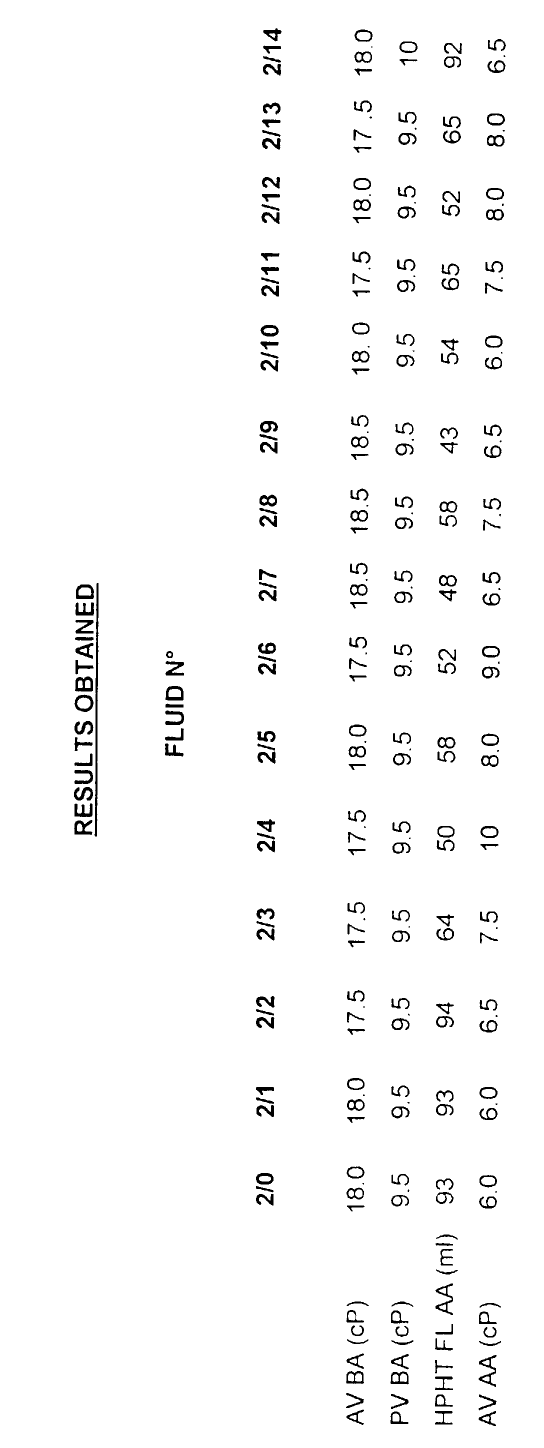

The above results show that addition of different types of APG equal to 2.8% in weight with respect to the water soluble polymer (CEPAC REGULAR) can reduce the HPHT filtrate after aging in variable proportions up to 53%. This effect is not present with APG having chains C1 and C4.

The fluid n° 2/14 shows that it is not possible to obtain good HPHT values when instead of adding APG the water soluble polymer is increased by the same quantity.

### EXAMPLE 3

Two water well fluids have been prepared, respectively without and with APG, as reported below.

| **FLUIDS COMPOSITION** | | |
|---|---|---|
| **COMPONENTS (g)** | **FLUID N° 3/1** | **FLUID N° 3/2** |
| FRESH WATER | 400 | 400 |
| KCl | 20 | 20 |
| CEPAC REGULAR | 1.15 | 1.15 |
| IDVIS | 0.80 | 0.80 |
| C618 | - | 0.80 |
| BARITE | 120 | 120 |

| **RESULTS OBTAINED** | | |
|---|---|---|
| | **FLUID N° 3/1** | **FLUID N° 3/2** |
| API FLc (ml/l) | 12 | 10 |
| AV BA (cP) | 21 | 24 |
| PV BA (cP) | 10 | 12 |
| YP BA (lb/100 ft²) | 22 | 24 |
| HPHT FL AA (ml) | 100 | 52 |
| AV AA (cP) | 8.5 | 14.5 |
| PV AA (cP) | 6.0 | 9.5 |
| YP AA (lb/100 ft²) | 5.0 | 10.0 |
| DENSITY (g/cm³) | 1.25 | 1.25 |

The results show that the addition of APG to the fluid produces sharp reduction of HPHT filtrate, improvement in the corrected API filtrate and in the rheology after aging.

### EXAMPLE 4

Two fluids have been prepared at high density and containing drill solids as reported below and the reduction of HPHT FL AA and the viscosity variation due to an APG addition have been determined.

| **FLUIDS COMPOSITION** | | |
|---|---|---|
| **COMPONENTS (g)** | **FLUID N° 4/1** | **FLUID N° 4/2** |
| SYNTHETIC SEA WATER (ml) | 350 | 350 |
| KCI | 10.5 | 10.5 |
| CEPAC REGULAR | 4 | 4 |
| MgO | 1 | 1 |
| KAOLIN | 10 | 10 |
| SODIUM BENTONITE | 10 | 10 |
| BARITE | 300 | 300 |
| CaCO₃ | 10 | 10 |
| C1618 | - | 1 |

| **RESULTS OBTAINED** | | |
|---|---|---|
| | **FLUID N° 4/1** | **FLUID N° 4/2** |
| PV BA (cP) | 40 | 40 |
| YP BA (lb/100 ft²) | 150 | 155 |
| PV AA (cP) | 31 | 46 |
| YP AA (lb/100 ft²) | 4 | 12 |
| HPHT FL AA (ml) | 25.4 | 13.6 |
| DENSITY (g/cm³) | 1.6 | 1.6 |

The results show that the addition of 25% in weight of an APG to CEPAC REGULAR improves the fluid resistance to thermal aging, limiting decrease in YP and with HPHT AA filtrate values halved.

### EXAMPLE 5

A dispersed bentonite fluid has been prepared in presence of contaminants and the improvement of the HPHT AA filtrate loss due to an addition of APG has been verified.

| **FLUIDS COMPOSITION** | | |
|---|---|---|
| **COMPONENTS(g)** | **FLUID N° 5/1** | **FLUID N° 5/2** |
| FRESH WATER | 380 | 380 |
| SODIUM BENTONITE | 19 | 19 |
| Q-BROXIN | 0.38 | 0.38 |
| CARBOCEL LV | 2 | 2 |
| CARBOCEL EHV | 0.6 | 0.6 |
| BARITE | 100 | 100 |
| KAOLIN | 20 | 20 |
| CaCO₃ | 10 | 10 |
| C1618 | - | 1.30 |

| **RESULTS OBTAINED** | | |
|---|---|---|
| | **FLUID N° 5/1** | **FLUID N° 5/2** |
| AV AA (cP) | 23.5 | 42.5 |
| PV AA (cP) | 16 | 26 |
| YP AA (lb/100 ft²) | 15 | 33 |
| HPHT FL AA (ml) | 22.6 | 16 |

The results show that the addition of an alkyl(poly)glycoside improves both the HPHT AA filtrate and the fluid rheology after aging.

### EXAMPLE 6

Fluids with increasing concentrations of APG have been prepared and the effect on the API filtrate loss corrected has been determined.

| **FLUIDS COMPOSITION** | | | | |
|---|---|---|---|---|
| **COMPONENTS (g)** | **FLUID N°** | | | |
| | **6/1** | **6/2** | **6/3** | **6/4** |
| FRESH WATER | 400 | 400 | 400 | 400 |
| KCI | 20 | 20 | 20 | 20 |
| IDVIS | 0.80 | 0.80 | 0 80 | 0.80 |
| LAMPAC EXLO | 0.80 | 0.80 | 0.80 | 0.80 |
| C1618 | - | 0.032 | 0.064 | 0.80 |
| BARITE | 12 | 12 | 12 | 12 |

| **RESULTS OBTAINED** | | | | |
|---|---|---|---|---|
| | **FLUID N°** | | | |
| | **6/1** | **6/2** | **6/3** | **6/4** |
| AV BA (cP) | 7.5 | 7.5 | 8.0 | 11.0 |
| API FLc (ml) | 15.4 | 11 | 10 | 4 |

The results show the effect of the APG quantity added on the fluid loss reduction.

### EXAMPLE 7

Fluids with PHPA have been prepared as reported below and the API filtrate corrected has been optimized.

| **FLUIDS COMPOSITION** | | | |
|---|---|---|---|
| **COMPONENTS (g)** | **FLUID N°** | | |
| | **7/1** | **7/2** | **7/3** |
| FRESH WATER | 400 | 400 | 400 |
| SODIUM BENTONITE | 8 | 8 | 8 |
| CARBOCEL LV | 0.80 | 0.40 | 0.80 |
| C1618 | - | 0.40 | 0.40 |
| POLY-PLUS | 2 | 2 | 1.6 |

| **RESULTS OBTAINED** | | | |
|---|---|---|---|
| | **FLUID N°** | | |
| | **7/1** | **7/2** | **7/3** |
| AV BA (cP) | 20 | 20 | 16.5 |
| PV BA (cP) | 15 | 15 | 12 |
| YP BA (lb/100 ft²) | 10 | 10 | 9 |
| API FLc (ml) | 13.2 | 10.8 | 9.6 |

In the above mentioned example, the optimization of the API fluid loss corrected is obtained by adding APG.

### EXAMPLE 8

A range of fluids for top hole has been prepared in order to study the action of APG addition on the API filtrate corrected loss of these fluids.

| **FLUIDS COMPOSITION** | | | | |
|---|---|---|---|---|
| **COMPONENTS (g)** | **FLUID N°** | | | |
| | **8/1** | **8/2** | **8/3** | **8/4** |
| FRESH WATER | 400 | 400 | 400 | 400 |
| SODIUM BENTONITE | 8 | 8 | 8 | 8 |
| LAMGUM 200 | 3.2 | 3.2 | 3.2 | 3.2 |
| C1618 | - | 0.13 | 0.32 | 0.96 |

| **RESULTS OBTAINED** | | | | |
|---|---|---|---|---|
| | **FLUID N°** | | | |
| | **8/1** | **8/2** | **8/3** | **8/4** |
| AV BA (cP) | 44.5 | 45 | 47 | 49 |
| PV BA (cP) | 16 | 16 | 16 | 16 |
| YP BA (lb/100 ft²) | 57 | 58 | 62 | 66 |
| API FLc (ml) | 10.9 | 9 | 6.4 | 4.2 |

The results show that in the above mentioned fluids the fluid loss reduction is proportional to the APG quantity added.

### EXAMPLE 9

The fluids reported below have been prepared in order to verify the variation in rheology and the corrected API filtrate loss of the Visplex system on addition of APG.

| **FLUIDS COMPOSITION** | | |
|---|---|---|
| **COMPONENTS (g)** | **FLUID N° 9/1** | **FLUID N° 9/2** |
| FRESH WATER | 184.3 | 184.3 |
| SODIUM BENTONITE | 15.8 | 15.8 |
| SEA WATER | 123 | 123 |
| VISPLEX | 1.5 | 1.5 |
| FLOPLEX | 3.5 | 3.5 |
| C1618 | - | 0. 50 |
| BARITE | 20 | 20 |

| **RESULTS OBTAINED** | | |
|---|---|---|
| | **FLUID N° 9/1** | **FLUID N° 9/2** |
| PV BA (cP) | 12 | 13 |
| YP BA (lb/100 ft²) | 30 | 36 |
| API FLc (ml) | 10.4 | 8.8 |

The results show that the APG addition to the Visplex system improves the rheology and the filtrate.

### EXAMPLE 10

Water soluble polymer based fluids have been prepared containing respectively APG with chain C₁₆-C₁₈, insoluble in water and, in comparison, APG with methyl and butyl, water soluble.

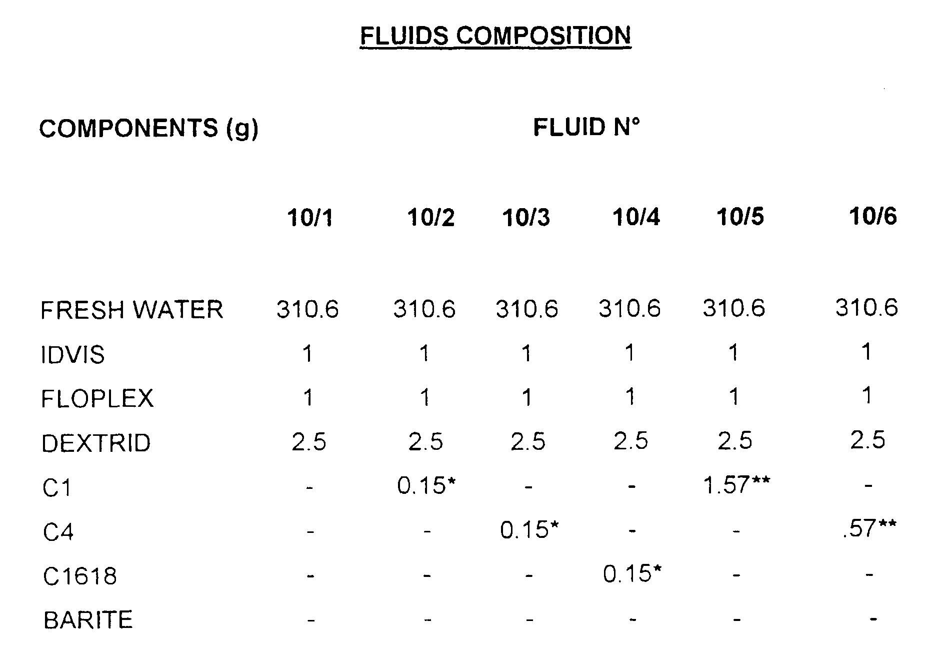

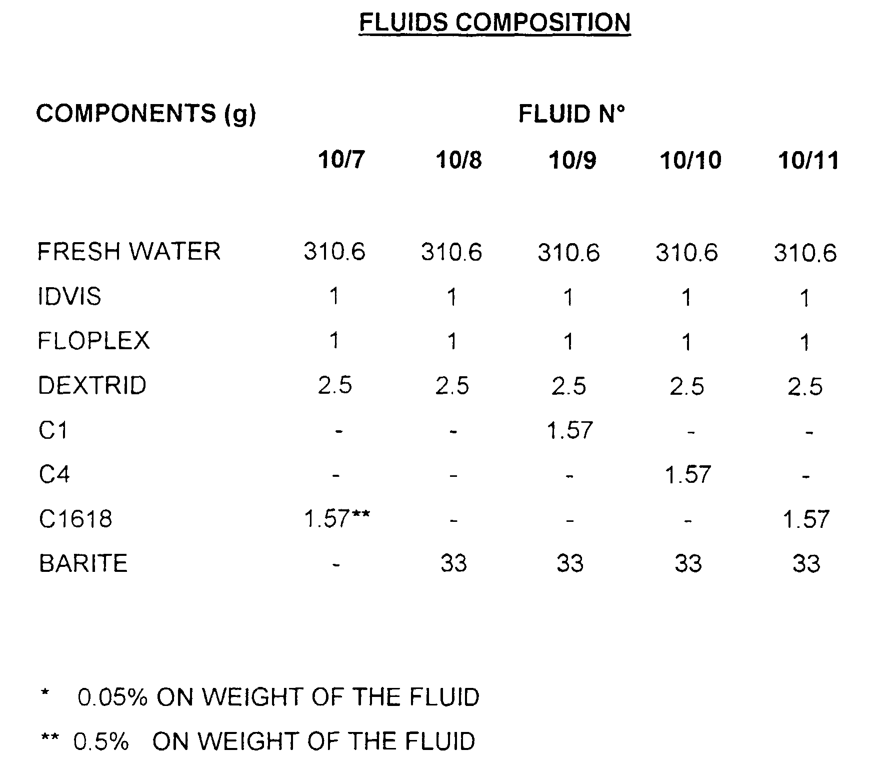

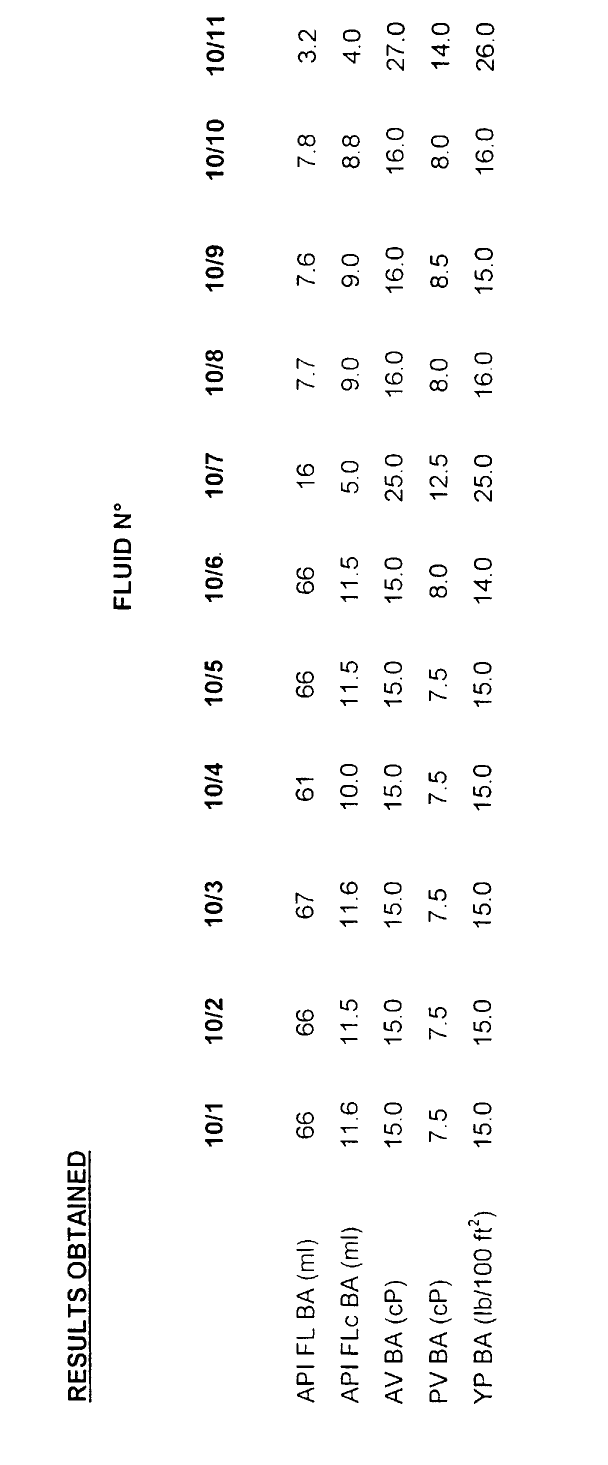

The results show that at the concentrations described in the example the APG with chain C₁₆-C₁₈ improves the fluid loss. No effect noted with methyl glucoside and butyl glucoside.

### EXAMPLE 11

The fluids reported below have been prepared in order to verify the variations due to the addition of APG in a silicate system.

| **FLUIDS COMPOSITION** | | | |
|---|---|---|---|
| **COMPONENTS (g)** | **FLUID N°** | | |
| | **11/1** | **11/2** | **11/3** |
| FRESH WATER | 350 | 350 | 350 |
| KCI | 15 | 15 | 15 |
| IDVIS | 1 | 1 | 1 |
| LAMPAC REGULAR | 1.5 | 1.5 | 1.5 |
| DEXTRID | 4 | 4 | 4 |
| NaOH | 0.5 | 0.5 | 0.5 |
| SODA ASH | 0.25 | 0.25 | 0.25 |
| Na SILICATE (% V/V) | 5 | 5 | 5 |
| C1618 | - | 0.15 | 0.65 |
| BARITE | 12 | 12 | 12 |

| **RESULTS OBTAINED** | | | |
|---|---|---|---|
| | **FLUID N°** | | |
| | **11/1** | **11/2** | **11/3** |
| API FL BA (ml) | 6.8 | 5.6 | 3.7 |
| AV BA (cP) | 31 | 34 | 37.5 |
| PV BA (cP) | 17.5 | 17 | 19.5 |
| YP BA (lb/100 ft²) | 27 | 34 | 36 |

The results show that the addition of APG in the silicate system improves the rheology and the filtrate.

## Claims

1. Water based well fluids having an improved fluid loss control and an improved resistance to the thermal aging, characterized by the fact that they contain both at least one water soluble polymer and at least one alkyl(poly)glycoside chosen among the compounds represented by the following formula (I):
R-O-(A-O)ₓ-(G)_{y}-(D)_{z} (I)
where:
O represents an oxygen atom;
R represents an alkyl or alkenyl group having from 8 to 26 C atoms, linear or branched, unsubstituted or hydroxy substituted;
A represents an alkylene group having from 2 to 4 C atoms, linear or branched, or a bifunctional residue of a polyalcohol remaining after removing 2 hydroxyl groups on any 2 carbon atoms;
G represents a saccharide residue remaining after removing z hydrogen atoms from all the non glycosidic hydroxyl groups and removing the glycosidic hydroxyl group of a reducing sugar made of hexoses or/and pentoses, bound to A-O or to R-O (in the case x=0) through an o-glycosidic ether bond;
D represents an acyl residue of an organic acid, bound to an oxygen atom of the residue (G)_{Y} having formula 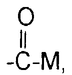 where M represents an alkyl or alkenyl chain having from 1 to 18 C atoms, linear or branched, unsubstituted or substituted by one or more groups such as OH, COOMt, SO₃Mt, -OPO₃Mt₂, NH₂, NR¹R², where R¹ and R² can be the same or different and represent alkyl chains having from 1 to 4 C atoms, linear or branched and Mt represents one hydrogen atom or a cation selected from Li, Na, K, Cs, Ca, Mg, Fe, NH₄+;
x is a number from 0 to 10 which represents the average condensation degree of A, when A represents an alkylene group and is equal to 1 when A represents a bifunctional residue of a polyalcohol remaining after removing 2 hydroxyl groups; y is a number from 1 to 10 which represents the average condensation degree of G;
z is a number from 0 to 10 which represents the average esterification degree of (G)_{Y}.

2. Well fluids according to the claim 1, characterized by the fact that said water soluble polymer is chosen in the group composed by cellulose ethers, polyanionic celluloses, starches, starch derivatives, polygalactomannans and derivatives thereof, biopolymers, homopolymers of acrylic acid, of acrylamide, of 2-acrylamido-2-methylpropanesulphonic acid and copolymers among these ones or of these ones with other acrylic or vinyl monomers and their water soluble salts.

3. Well fluids according to the claim 1, characterized by the fact that in said formula (I):
R represents a linear alkyl group having from 10 to 26 C atoms, unsubstituted or hydroxy substituted;
x = 0, or = 1 when A is a bifunctional residue of sorbitol remaining after removing 2 hydroxyl groups;
G represents a glucose residue remaining after removing 1 hydrogen atom from the non glycosidic hydroxyl groups and removing the glycosidic hydroxyl group;
y is a number from 1 to 3;
z = 0, or = 1 when
D = CO(CHOH)₂COOMt or
D=COCH₂C(OH)(COOMt)CH₂COOMt or
D = COCH₂CH(SO₃Mt)COOMt or
D = COCH(SO₃Mt)CH₂COOMt, with Mt = H or Na.

4. Well fluids according to the claim 1, characterized by the fact that in said formula (I):
x = 0, G is a glucose residue, y = 1-2, z = 0 and R is linear saturated not substituted alkyl having from 10 to 26 C atoms.

5. Well fluids according to the claim 1, characterized by the fact that in said formula (I):
x = 0, G is a glucose residue, y = 1-2, z = 1 and D is a monoacyl residue of a bi- or tri-carboxylic acid, optionally sulphonated, in acid form or salified with Na and R is a linear saturated not substituted alkyl group having from 10 to 26 C atoms.

6. Well fluids according to the claim 1, characterized by the fact that in said formula (I):
R is a linear alkyl group having from 10 to 18 C atoms substituted with hydroxyl group in position 2, A is a sorbitol bifunctional residue, x = 1, G is a glucose residue, y = 1-2, z = 0.

7. Well fluids according to the claim 1, characterized by the fact that they contain an alkyl(poly)glycoside having alkyl chains from 10 to 26 C atoms and a water soluble polymer chosen among carboxymethylcellulose, polyanionic cellulose, guar and xanthan gums.

8. Well fluids according to the claim 1, characterized by the fact that said alkyl(poly)glycoside is contained in quantity from 0.001 to 3% in weight with respect to the fluid, and said water soluble polymer is contained in quantity from 0.02 to 5% in weight with respect to the fluid.

9. Well fluids according to the claim 1, characterized by the fact that said alkyl(poly)glycoside is contained in quantity from 0.001 to 3% in weight with respect to the fluid, and said water soluble polymer is contained in quantity from 0.05 to 3% in weight with respect to the fluid.

10. Procedure for the preparation of well fluids as described in claim 1, characterized by the fact that to a water composition comprising thickening agents, viscosifiers, rheological modifiers, dispersants, emulsifiers, lubricants, defoamers, biocides, pH modifiers and corrosion inhibitors, are added at least one alkyl(poly)glycoside having formula (I) in quantity from 0.001 to 3% in weight with respect to the fluid and at least one water soluble polymer in quantity from 0.02 to 5% in weight with respect to the fluid.

11. Procedure according to the claim 10, characterized by the fact that said alkyl(poly)glycoside is added directly to said water composition as is or in the form of solution or suspension.

12. Procedure according to claim 10, characterized by the fact that said alkyl(poly)glycoside is added to said water composition in form of a mix with one of said agents or with said water soluble polymer.

13. Use of well fluids as described in claim 1, in drilling, completion, work-over, milling stimulation, fracturing and spotting fluids operations, concerning hydrocarbons or water beds research, geognostic drilling and civil engineering operations.

## Patentansprüche

1. Bohrflüssigkeiten auf Wasserbasis, die eine verbesserte Flüssigkeitsverlust-Kontrolle und eine verbesserte thermische Alterungsbeständigkeit aufweisen, dadurch gekennzeichnet, daß sie sowohl mindestens ein wasserlösliches Polymer als auch mindestens ein Alkyl(poly)glycosid enthalten, ausgewählt aus den Verbindungen mit der folgenden Formel (I):
R-O-(A-O)ₓ-(G)_{y}-(D)_{z} (I)
worin bedeuten:
O ein Sauerstoffatom:
R eine lineare oder verzweigte, unsubstituierte oder Hydroxy-substituierte Alkyl- oder Alkenylgruppe mit 8 bis 26 Kohlenstoffatomen;
A eine lineare oder verzweigte Alkylengruppe mit 2 bis 4 Kohlenstoffatomen oder einen bifunktionellen Rest eines Polyalkohols, wie er nach der Entfernung von zwei Hydroxylgruppen an beliebigen 2 Kohlenstoffatomen zurückbleibt;
G einen Saccharid-Rest, wie er nach der Entfernung von z Wasserstoffatomen aus allen nicht-glycosidischen Hydroxylgruppen und der Entfernung der glycosidischen Hydroxylgruppe eines reduzierenden Zuckers, aufgebaut aus Hexosen und/oder Pentosen, zurückbleibt, der über eine o-glycosidische Etherbindung an A-O oder an R-O (für den Fall, daß x = 0) gebunden ist;
D einen Acyl-Rest einer organischen Säure, der an ein Sauerstoffatom des Restes (G)_{y} gebunden ist und die Formel 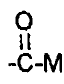 hat, worin M steht für eine lineare oder verzweigte Alkyl- oder Alkenylkette mit 1 bis 18 Kohlenstoffatomen, die unsubstituiert oder substituiert ist durch eine oder mehrere Gruppen wie OH, COOMt, SO₃Mt, -OPO₃Mt₂, NH₂, NR¹R², worin R¹ und R² gleich oder verschieden sein können und lineare oder verzweigte Alkylketten mit 1 bis 4 Kohlenstoffatomen und Mt ein Wasserstoffatom oder ein Kation, ausgewählt aus Li, Na, K, Cs, Ca, Mg, Fe, NH₄⁺, darstellen;
x eine Zahl von 0 bis 10, die den durchschnittlichen Kondensationsgrad von A repräsentiert, wenn A eine Alkylengruppe darstellt, und die Zahl 1, wenn A einen bifunktionellen Rest eines Polyalkohols, wie er nach der Entfernung von zwei Hydroxylgruppen zurückbleibt, darstellt;
y eine Zahl von 1 bis 10, die den durchschnittlichen Kondensationsgrad von G repräsentiert; und
z eine Zahl von 0 bis 10, die den durchschnittlichen Veresterungsgrad von (G)_{y} repräsentiert.

2. Bohrflüssigkeiten nach Anspruch 1, dadurch gekennzeichnet, daß das genannte wasserlösliche Polymer ausgewählt wird aus der Gruppe, die besteht aus Celluloseethern, polyanionischen Cellulosen, Stärken, Stärke-Derivaten, Polygalactomannanen und Derivaten davon, Biopolymeren, Homopolymeren von Acrylsäure, Acrylamid, 2-Acrylamido-2-methylpropansulfonsäure und Copolymeren zwischen diesen Monomeren oder Copolymeren dieser Monomeren mit anderen Acryl- oder Vinyl-Monomeren und ihren wasserlöslichen Salzen.

3. Bohrflüssigkeiten nach Anspruch 1, dadurch gekennzeichnet, daß in der genannten Formel (I) bedeuten:
R eine unsubstituierte oder Hydroxy-substituierte lineare Alkylgruppe mit 10 bis 26 Kohlenstoflatomen;
x die Zahl 0 oder 1, wenn A für einen bifunktionellen Rest von Sorbit steht, wie er nach der Entfernung von zwei Hydroxylgruppen zurückbleibt;
G einen Glucose-Rest, wie nach der Entfernung von einem Wasserstoffatom aus den nicht-glycosidischen Hydroxylgruppen und der Entfernung der glycosidischen Hydroxylgruppe zurückbleibt;
y eine Zahl von 1 bis 3;
z die Zahl 0, oder 1, wenn
D = CO(CHOH)₂COOMt oder
D = COCH₂C(OH)(COOMt)CH₂COOMt oder
D = COCH₂CH(SO₃Mt)COOMt oder
D = COCH(SO₃Mt)CH₂COOMt, wobei Mt = H oder Na.

4. Bohrflüssigkeiten nach Anspruch 1, dadurch gekennzeichnet, daß in der genannten Formel (I) bedeuten:
x die Zahl 0,
G einen Glucose-Rest,
y eine Zahl von 1 bis 2,
z die Zahl 0 und
R eine lineare, gesättigte, unsubstituierte Alkylgruppe mit 10 bis 26 Kohlenstoffatomen.

5. Bohrflüssigkeiten nach Anspruch 1, dadurch gekennzeichnet, daß in der genannten Formel (I) bedeuten:
x die Zahl 0,
G einen Glucose-Rest,
y eine Zahl von 1 bis 2,
z die Zahl 1 und
D einen Monoacyl-Rest einer Bi- oder Tricarbonsäure, die gegebenenfalls sulfoniert ist, in der Säureform oder in der Na-Salz-Form und
R eine lineare; gesättigte; unsubstituierte Alkylgruppe mit 10 bis 26 Kohlenstoffatomen.

6. Bohrflüssigkeiten nach Anspruch 1, dadurch gekennzeichnet, daß in der genannten Formel (I) bedeuten:
R eine lineare Alkylgruppe mit 10 bis 18 Kohlenstoffatomen, die in der 2-Position durch eine Hydroxylgruppe substituiert ist,
A einen bifunktionellen Sorbit-Rest,
x die Zahl 1,
G einen Glucose-Rest,
y eine Zahl von 1 bis 2 und
z die Zahl 0.

7. Bohrflüssigkeiten nach Anspruch 1, dadurch gekennzeichnet, daß sie ein Alkyl(poly)glycosid, das Alkylketten mit 10 bis 26 Kohlenstoffatomen aufweist, und ein wasserlösliches Polymer, ausgewählt aus Carboxymethylcellulose, polyanionischer Cellulose, Guargummi und Xanthangummi, enthalten.

8. Bohrflüssigkeiten nach Anspruch 1, dadurch gekennzeichnet, daß das genannte Alkyl(poly)glycosid in einer Menge von 0,001 bis 3 Gew.-%, bezogen auf die Flüssigkeit, enthalten ist; und daß das genannte wasserlösliche Polymer in einer Menge von 0,02 bis 5 Gew.-%, bezogen auf die Flüssigkeit, enthalten ist.

9. Bohrflüssigkeiten nach Anspruch 1, dadurch gekennzeichnet, daß das genannte Alkyl(poly)glycosid in einer Menge von 0,001 bis 3 Gew.-%, bezogen auf die Flüssigkeit, enthalten ist; und daß das genannte wasserlösliche Polymer in einer Menge von 0,05 bis 3 Gew.-%, bezogen auf die Flüssigkeit, enthalten ist.

10. Verfahren zur Herstellung von Bohrflüssigkeiten, wie sie in Anspruch 1 beschrieben sind, dadurch gekennzeichnet, daß einer wäßrigen Zusammensetzung, die Verdickungsmittel, Viskositäts-Modifizierungsmittel, rheologische Modifizierungsmittel, Dispergiermittel, Emulgiermittel, Gleit- bzw. Schmiermittel, Antischaummittel, Biozide, pH-Modifizierungsmittel und Korrosionsinhibitoren umfaßt, mindestens ein Alkyl(poly)glycosid der Formel (I) in einer Menge von 0,001 bis 3 Gew.-%, bezogen auf die Flüssigkeit, und mindestens ein wasserlösliches Polymer in einer Menge von 0,02 bis 5 Gew.-%, bezogen auf die Flüssigkeit, zugesetzt werden.

11. Verfahren nach Anspruch 10, dadurch gekennzeichnet, daß das genannte Alkyl(poly)glycosid direkt der genannten wäßrigen Zusammensetzung, wie sie vorliegt, oder in Form einer Lösung oder Suspension zugesetzt wird.

12. Verfahren nach Anspruch 10, dadurch gekennzeichnet, daß das genannte Alkyl(poly)glycosid der genannten wäßrigen Zusammensetzung in Form einer Mischung mit einem der genannten Agentien oder mit dem genannten wasserlöslichen Polymer zugesetzt wird.

13. Verwendung der Bohrflüssigkeiten, wie sie in Anspruch 1 beschrieben sind, bei Bohr-, Komplettierungs-, Work-over-, Mahlstimulations-, Frakturierungs- und Spottingfluid-Arbeiten bei der Erforschung von Kohlenwasserstoff- oder Wasser-Lagerstätten, bei geologischen Bohrungs- und Tiefbauarbeiten.

## Revendications

1. Fluides aqueux pour puits ayant un meilleur contrôle de la perte de fluide et une meilleure résistance au vieillissement thermique, caractérisés en ce qu'ils contiennent à la fois, au moins un polymère hydrosoluble et au moins un alkyl(poly)-glucoside choisi parmi les composés représentés par la formule suivante (I) :
R-O-(A-O)ₓ-(G)_{y}-(D)_{Z} (I)
dans laquelle :
O représente un atome d'oxygène ;
R représente un radical alkyle ou alcényle, linéaire ou ramifié, ayant de 8 à 26 atomes de carbone, non substitué ou substitué par des groupes hydroxyles;
A représente un groupe alkyle linéaire ou ramifié ayant de 2 à 4 atomes de carbone, ou un résidu bi-fonctionnel d'un polyalcool, résultant de l'élimination de deux groupes hydroxyles de n'importe quel atome de carbone dudit polyalcool;
G représente un résidu de saccharide résultant de l'élimination de z atomes d'hydrogène de tous les groupes hydroxyles non glycosidiques et de l'élimination du groupe hydroxyle glycosidique d'un sucre réducteur à base d'hexoses et/ou de pentoses, ledit saccharide étant lié à A-O ou à R-O (dans le cas où x=0), par une liaison éther o-glycosidique ;
D représente le radical acyle d'un acide organique, lié à un atome d'oxygène du résidu (G)y, ayant pour formule 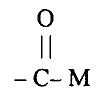 où M représente une chaîne alkyle ou alcényle, ayant de 1 à 18 atomes de carbone, linéaire ou ramifiée, non substituée ou substituée par un ou plusieurs groupes tels que OH, COOMt, SO₃Mt, OPO₃Mt₂, NH₂, NR¹R² où R¹ et R² sont identiques ou différents et représentent une chaîne alkyle ayant de 1 à 4 atomes de C, linéaire ou ramifiée et Mt représente un atome d'hydrogène ou un cation choisi parmi Li, Na, K, Cs, Ca, Mg, Fe, NH₄⁺ ;
x est un nombre compris entre 0 et 10 qui représente le degré moyen de condensation de A, lorsque A représente un groupe alkylène et est égal à 1 lorsque A représente un résidu bi-fonctionnel de polyalcool résultant de l'élimination de deux groupes hydroxyles ;
y est un nombre de 1 à 10 qui représente le degré moyen de condensation de G;
z est un nombre de 1 à 10 qui représente le degré moyen d'estérification de (G)_{y} .

2. Fluides pour puits selon la revendication 1, caractérisés en ce que ledit polymère hydrosoluble est choisi dans le groupe constitué par les éthers de cellulose, les celluloses poly-anioniques, les amidons, les dérivés de l'amidon, les polygalactomananes et leurs dérivés, les bio-polymères, les homo-polymères de l'acide acrylique, de l'acrylamide, de l'acide 2-acrylamido-2-méthyl-propane-sulfonique et les copolymères de ces derniers entre eux ou avec d'autres monomères acryliques ou vinyliques, et leurs sels hydrosolubles.

3. Fluides pour puits selon la revendication 1, caractérisés en ce que, dans ladite formule (I) :
R représente un groupe alkyle de 10 à 26 atomes de C, non substitué ou substitué par des hydroxyles ;
x = 0, ou = 1 lorsque A est le résidu bifonctionnel du sorbitol résultant de l'élimination de deux groupes hydroxyles ;
G représente un résidu de glucose résultant de l'élimination d'un atome d'hydrogène des groupes hydroxyles non glycosidiques et de l'élimination du groupe hydroxyle glycosidique ;
y est un nombre compris entre 1 à 3 ;
z = 0, ou = 1 lorsque D = CO(CHOH)₂COOMt ou
D = COCH₂C(OH)(COOMt) CH₂COOMt ou
D = COCH₂CH(SO₃Mt)COOMt ou D= COCH(SO₃Mt)CH₂COOMt avec Mt = H ou Na.

4. Fluides pour puits selon la revendication 1, caractérisés en ce que, dans la dite formule (I) :
x = 0, G est un résidu de glucose, y = 1-2, z = 0 et R est un alkyle linéaire saturé non substitué ayant de 10 à 26 atomes de C.

5. Fluides pour puits selon la revendication 1, caractérisés en ce que, dans la dite formule (I) :
x = 0, G est un résidu de glucose, y = 1-2, z = 1 et D est un résidu monoacyle d'un acide bi- ou tri-carboxylique, éventuellement sulfoné, sous forme acide ou salifiée avec Na, et R est un groupe alkyle linéaire saturé non substitué, ayant de 10 à 26 atomes de C.

6. Fluides pour puits selon la revendication 1, caractérisés en ce que, dans ladite formule (I) :
R est un groupe alkyle linéaire ayant de 10 à 18 atomes de C, substitué par un groupe hydroxyle en position 2, A est un résidu bifonctionnel du sorbitol, x = 1, G est un résidu du glucose, y = 1-2, z=0.

7. Fluides pour puits selon la revendication 1, caractérisés en ce qu'ils comprennent un alkyl-(poly)-glucoside ayant des chaînes alkyles de 10 à 26 atomes de C et un polymère hydrosoluble choisi parmi la carboxy-méthyl-cellulose, la cellulose poly-anionique, et les gommes de guar et de xanthane.

8. Fluides pour puits selon la revendication 1, caractérisés en ce que la teneur en alkyl-(poly)-glucoside est comprise entre 0,001 et 3 % en poids par rapport au fluide et que la teneur en polymère hydrosoluble est comprise entre 0,02 et 5 % en poids par rapport au fluide.

9. Fluides pour puits selon la revendication 1, caractérisés en ce que la teneur en alkyl-(poly)-glucoside est comprise entre 0,001 et 3 % en poids par rapport au fluide et que la teneur en polymère hydrosoluble est comprise entre 0,05 et 3 % en poids par rapport au fluide.

10. Procédé pour la préparation des fluides pour puits définis selon la revendication 1, caractérisé en ce qu'à une composition aqueuse comprenant des agents densifiants, des agents de viscosité, des modificateurs de rhéologie, des dispersants, des émulsionnants, des lubrifiants, des antimousses, des biocides, des agents modifiant le pH et des inhibiteurs de corrosion, sont ajoutés au moins un alkyl(poly)-glucoside ayant la formule (I), à raison de 0,001 à 3 % en poids par rapport au fluide et au moins un polymère hydrosoluble à raison de 0,02 à 5 % en poids par rapport au fluide.

11. Procédé selon la revendication 10, caractérisé en ce que ledit alkyl-(poly)-glucoside est ajouté à la dite composition aqueuse directement tel quel ou sous forme de solution ou de suspension.

12. Procédé selon la revendication 10, caractérisé en ce que ledit alkyl-(poly)-glucoside est ajouté à la composition aqueuse sous forme de mélange avec un desdits agents ou avec ledit polymère hydrosoluble.

13. Utilisation des fluides pour puits tels que définis dans la revendication 1, dans les opérations de forage, de compléments, de reconditionnements des puits, de stimulation du broyage; dans les opérations de fracturation et de spotting des fluides, relatives à la recherche d'hydrocarbures ou de nappes aquifères, aux prospections géognostiques et aux opérations d'ingénierie civile.
